# FASCICULE DE BREVET EUROPEEN

(11) **EP 1 397 121 B1**
(45) Date de publication et mention de la délivrance du brevet: **27.09.2006**
(21) Numéro de dépôt: 02740849.1
(22) Date de dépôt: 31.05.2002
(51) Int. Cl.: A61K 8/19, A61K 8/41, A61Q 5/00, A61Q 19/00

(54) **COMPOSITION COSMETIQUE COMPRENANT DES PARTICULES DE CARBONATE DE CALCIUM ET DES AGENTS DE CONDITIONNEMENT**
KOSMETISCHE ZUSAMMENSETZUNG, DIE CALCIUMKARBONATTEILCHEN UND KONDITIONIERUNGSMITTEL ENTHÄLT
COSMETIC COMPOSITION COMPRISING CALCIUM CARBONATE PARTICLES AND CONDITIONING AGENTS

(30) Priorité: 31.05.2001 FR 0107152
(43) Date de publication de la demande: 17.03.2004
(73) Titulaire: L'ORÉAL, 75008 Paris (FR)
(72) Inventeur: PERRON, Béatrice, F-78350 Jouy En Josas (FR); RESTLE, Serge, F-95390 Saint-Prix (FR); GIROUD, Franck, F-92110 Clichy (FR); SAMAIN, Henri, F-91570 Bièvres (FR)
(74) Mandataire: Le Blainvaux Bellegarde, Françoise
(86) Numéro de dépôt international: PCT/FR2002/001850
(87) Numéro de publication internationale: WO 2002/096385

(56) Documents cités:
- WO-A-99/25312
- DE-A- 19 946 784
- US-A- 3 958 581
- US-A- 4 895 722
- US-A- 5 334 376
- PATENT ABSTRACTS OF JAPAN vol. 1999, no. 13, 30 novembre 1999 (1999-11-30) & JP 11 228332 A (SUNSTAR INC), 24 août 1999 (1999-08-24)
- PATENT ABSTRACTS OF JAPAN vol. 014, no. 109 (C-0695), 28 février 1990 (1990-02-28) & JP 01 313415 A (HOOU KK), 18 décembre 1989 (1989-12-18)

## Description

La présente invention est relative à une composition cosmétique, notamment capillaire, contenant, dans un milieu cosmétiquement acceptable, des particules de carbonate de calcium et au moins un agent de conditionnement. Elle vise également un procédé de traitement cosmétique des cheveux comprenant l'application de cette composition ainsi que son utilisation en tant que produit capillaire rincé.

Pour le nettoyage et/ou le lavage des cheveux, l'utilisation de compositions capillaires détergentes (ou shampooings), à base essentiellement d'agents tensioactifs classiques de types notamment anionique, non-ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés de silicone, qui confèrent en effet aux cheveux lavés, secs ou mouillés, une facilité de démêlage et une douceur nettement accrue par rapport à ce qui peut être obtenu avec les compositions nettoyantes correspondantes qui en sont exemptes.

Cependant, ces avantages cosmétiques s'accompagnent malheureusement également, sur cheveux séchés, de certains effets cosmétiques jugés indésirables, à savoir un alourdissement de la coiffure, un manque de lissage.

En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion entre les fibres affectant le coiffage. Ces inconvénients sont accentués dans le cas de cheveux fins, qui manquent de nervosité et de volume.

Il a déjà été proposé l'utilisation de particules dans des compositions rincées, de façon à améliorer le toucher et l'aspect des cheveux. A titre d'illustration, le brevet US 5 334 376 propose l'ajout de particules de sulfate de barium dans des compositions de conditionnement des cheveux contenant une silicone, un alcool gras et une amide.

Dans la demande de brevet DE 199 46 784, il a également été proposé l'utilisation de particules de différents oxydes, hydroxydes, carbonates, silicates ou phosphates, dans des compositions capillaires, pour réduire l'aspect gras des cheveux. Il est prévu, en toutes généralités, d'associer ces particules aux ingrédients classiques des shampooings.

Toutefois, et malgré les progrès réalisés récemment dans le domaine des produits capillaires rincés et notamment des shampooings, ces derniers ne donnent pas vraiment complètement satisfaction, de sorte qu'un fort besoin existe encore actuellement quant à pouvoir disposer de nouveaux produits présentant, au niveau de l'une ou de plusieurs de leurs propriétés, de meilleures performances.

La Demanderesse a découvert, de manière surprenante et inattendue, qu'en choisissant l'agent de conditionnement, associé avec des particules de carbonate de calcium, il était possible d'améliorer les résultats obtenus avec les produits cosmétiques, notamment capillaires rincés, en terme de propriétés cosmétiques et de mise en forme. En particulier, on apporte de la texture aux cheveux (sensation d'épaisseur accrue) et une meilleure tenue de la coiffure.

L'invention a pour objet une composition cosmétique, notamment capillaire, comprenant, dans un milieu cosmétiquement acceptable :
(a) des particules solides contenant au moins 10 % en poids de carbonate de calcium ;
(b) au moins un agent de conditionnement choisi parmi :
   (i)les tensioactifs cationiques choisis parmi les esters quaternaires, les diammonium quaternaires sauf ceux dérivés du diamino-propane, les imidazolinium et les amides,
   (ii)les silicones cationiques, et
   (iii) les polymères cationiques dont la densité de charge cationique est inférieure ou égale à 7 meq/g et, de préférence, supérieure ou égale à 0,05 meq/g.

Un autre objet de la présente invention consiste en un procédé de traitement cosmétique des cheveux mettant en oeuvre la composition selon l'invention.

L'invention a encore pour objet l'utilisation de la composition en cosmétique capillaire, notamment en application capillaire rincée.

D'autres objets, caractéristiques, aspects et avantages de l'invention apparaîtront encore plus clairement à la lecture de la description et des divers exemples qui suivent.

### (a) PARTICULES DE CARBONATE DE CALCIUM

Les particules contenant au moins 10 % en poids de carbonate de calcium présentent, de préférence, une taille primaire moyenne en nombre comprise entre 2 nm et 2 µm, plus préférentiellement entre 5 et 500 nm, et plus préférentiellement encore entre 10 et 250 nm.

Les particules selon l'invention peuvent, par exemple, avoir une forme quelconque, par exemple la forme de sphères, de paillettes, d'aiguilles, de plaquettes ou des formes totalement aléatoires.

Conformément à la présente invention, la particule peut être une particule massique formée entièrement de carbonate de calcium. Le carbonate de calcium peut également constituer totalement ou partiellement le coeur de la particule, ce dernier étant recouvert d'un autre constituant, comme par exemple, un oxyde, un silicate ou un métal. Le carbonate de calcium peut encore former uniquement le revêtement d'un substrat de constitution chimique différente, comme par exemple un oxyde, un silicate ou un métal.

Au sens de la présente invention, on entend par *"taille primaire de particule",* la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Dans le cas où les particules sont formées par du carbonate de calcium et d'autres charges, le carbonate de calcium se trouve à l'état libre et ne forme pas de liaisons chimiques avec les autres charges. Il s'agit alors d'un alliage entre le carbonate de calcium et d'autres charges, notamment avec des oxydes de métaux ou de métalloïdes, en particulier obtenu par fusion thermique de ces différents constituants.

Lorsque les particules contenant au moins 10 % en poids de carbonate de calcium comprennent, en outre, un oxyde de métal ou de métalloïde, celui-ci est notamment choisi parmi l'oxyde de silicium, de bore ou d'aluminium.

De préférence, les particules contiennent au moins 50 % en poids de carbonate de calcium, mieux encore au moins 70 % en poids, et les particules constituées à plus de 90 % en poids de carbonate de calcium sont particulièrement préférées selon la présente invention.

Plus avantageusement encore, les particules contenant au moins 10 % en poids de carbonate de calcium sont des particules de carbonate de calcium substantiellement pur.

Les particules contenant du carbonate de calcium selon l'invention sont, notamment, utilisées en une quantité comprise entre 0,01 % et 30 % en poids, et de préférence entre 0,05 % et 5 % en poids par rapport au poids total de la composition.

Le carbonate de calcium convenant dans les compositions de la présente invention peut être d'origine naturelle ou peut être d'origine synthétique. Dans ce dernier cas, il peut être obtenu à partir d'oxyde de calcium, de peroxyde de calcium, d'acétate ou d'éthylate de calcium.

La composition selon l'invention peut également contenir d'autres types de particules, par exemple, des particules d'oxyde de titane ou de zinc, d'aluminium.

### (b)AGENTS DE CONDITIONNEMENT

Les agents de conditionnement convenant à la mise en oeuvre de la présente invention sont notamment les suivants :

### (i) les tensioactifs cationiques choisis parmi les esters quaternaires, les diammonium quaternaires, les imidazolinium et les amides

### - les esters quaternaires

- les sels de diammonium quaternaire présentent avantageusement la formule (III) : 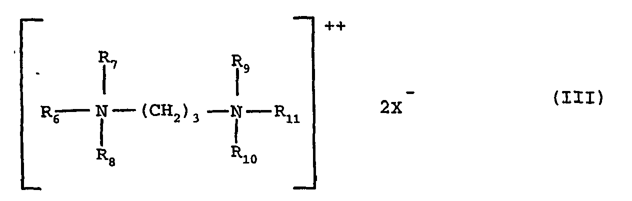 dans laquelle R₆ désigne un radical aliphatique comportant de 16 à 22 atomes de carbone, R₇, R₈, R₉, R₁₀, et R₁₁ sont choisis parmi l'hydrogène ou un radical alkyle comportant de 1 à 4 atomes de carbone, et X est un anion choisi dans le groupe des halogénures, acétates, phosphates, et sulfates.
   Les sels de diammonium quaternaire visés par la présente invention de sont pas dérivés du diamino-propane.
- les sels d'ammonium quaternaire de l'imidazolinium présentent avantageusement la formule (II) suivante : 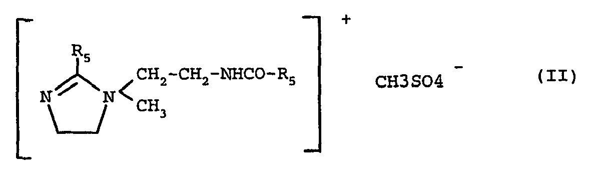 dans laquelle R₅ représente un mélange de radicaux alcényle et/ou alkyle comportant de 13 à 21 atomes de carbone et dérivés des acides gras du suif.
- les amides présentant la formule générale (I) suivante : 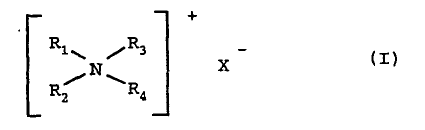 dans laquelle les radicaux R₁ à R₄, qui peuvent être identiques ou différents, représentent un radical alkylamide, comportant environ de 12 à environ 22 atomes de carbone; X est un anion choisi dans le groupe des halogénures, phosphates, acétates, lactates, alkylsulfates, alkyl-ou-alkylarylsulfonates.

### (ii)les silicones cationiques

De telles silicones cationiques comprennent notamment les polymères de formule (V):

(R₁₂)_{c} G_{3-c}-Si-(-OSi G₂)ₙ-[OSi G_{d} (R₁₂)_{2-d}]ₘ-O-Si G_{3-c}(R₁₂)_{c} (V)

dans laquelle
- G est choisi dans le groupe formé par H, OH, alkyle en C₁₋₈, et phényle, et désigne de préférence méthyle,
- a désigne 0 ou un nombre entier allant de 1 à 3, et de préférence est égal à 0,
- b désigne 0 ou 1, et de préférence est égal à 1,
- la somme (n+m) représente un nombre allant de 0 à 2000 et de préférence allant de 50 à 150, n pouvant désigner un nombre allant de 0 à 1999 et de préférence allant de 49 à 149 et m pouvant désigner un nombre allant de 1 à 2000 et de préférence allant de 1 à 10,
- R₁₂ est un radical monovalent de formule C_{q} H_{2q} L dans laquelle q = 2 à 8,
L étant choisi parmi les groupements :

- N(R₁₃)CH₂ - CH₂ - N(R₁₃)₂

- N(R₁₃)₂

- N^{⊕}(R₁₃)₃ A⁻

- N^{⊕}(R₁₃)CH₂ - CH₂ - N^{⊕}(R₁₃)H₂ A⁻

dans lesquels R₁₃ est choisi dans le groupe formé par H, phényle, benzyle, un radical hydrocarboné saturé, de préférence un radical alkyle comportant de 1 à 20 atomes de carbone, et A⁻ désigne un ion halogénure.

Ces polymères de formule (V) sont notamment des composés décrits dans le dictionnaire CTFA sous les dénomonations « Amodiméthicone » et « Triméthylsilylamodiméthicone ».

Une amodiméthicone, qui est ici plus particulièrement préférée, est celle vendue sous la dénomination commerciale « Emulsion cationique DC 939 » (ou DC 939) par la société Dow Corning, qui est une asssociation d'Amodiméthicone, de chlorure d'hexadécyltriméthylammonium et d'alcool tridécylique polyoxyéthyléné, en émulsion aqueuse à 36% d'Amodiméthicone.

Une triméthylsilylamodiméthicone plus particulièrement préférée est celle vendue par la société Dow Corning sous la dénomination « Dow Corning Q2 7224 ».

D'autres silicones cationiques pouvant encore être utilisées dans la composition selon la présente invention sont des polymères de formule (VII) : 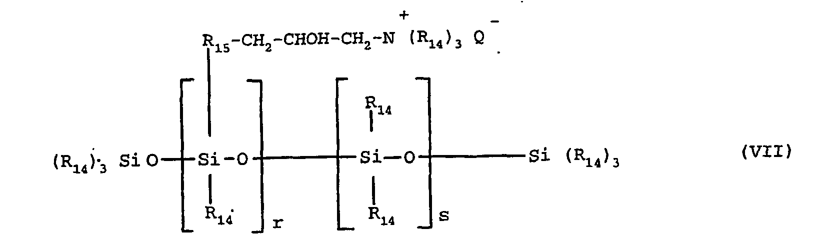 dans laquelle,
- R₁₄ désigne un radical hydrocarboné comportant de 1 à 18 atomes de carbone, en particulier un radical alkyle ou alkényle et de préférence méthyle,
- R₁₅ désigne un radical hydrocarboné, de préférence un radical alkylène comportant de 1 à 18 atomes de carbone ou un radical alkylèneoxy comportant de 1 à 18 atomes de carbone et de préférence de 1 à 8 atomes de carbone,
- Q⁻ désigne un ion halogénure et de préférence un ion chlorure,
- r représente une valeur statistique moyenne allant de 2 à 20 et de préférence de 2 à 8,
- s représente une valeur statistique moyenne allant de 20 à 200 et de préférence de 20 à 50.

Ces composés sont décrits plus en détail dans le brevet US- 4 185 087.

Un polymère de ce type plus particulièrement préféré est celui vendu par la société Union Carbide sous la dénomination « Ucar Silicone Ale 56 ».

De préférence, la silicone cationique est une choisie parmi les « Amodiméthicone » et « Triméthylsilylamodiméthicone ».

### (iii) les polymères cationiques dont la densité de charge cationique est inférieure ou égale à 7 meq/g et, de préférence, supérieure ou égale à 0,05meq/g

Les polymères cationiques utilisés conformément à l'invention ont en général un poids moléculaire moyen en masse d'au moins 5000, préférentiellement d'au moins 10.000 et inférieur à 10.000.000 et plus particulièrement allant de 100.000 à 2.000.000. Ils ont en général des motifs contenant un atome d'azote tels que des motifs ammoniums quaternaires ou amino ou leurs mélanges. Leur densité de charge cationique est inférieure ou égale à 7 meq/g et, de préférence, supérieure ou égale à 0,05 meq/g, plus préférentiellement, cette densité de charge est comprise entre 0,5 et 7 meq/g. La densité de charge peut être déterminée selon la méthode Kjeldahl. Elle correspond en général à un pH de l'ordre de 3 à 9.

Parmi les polymères cationiques utilisables selon l'invention, on peut citer les copolymères de monomères vinyliques ayant des fonctions amines ou ammoniums quaternaires avec des monomères à insaturation éthylénique hydrosolubles tels que l'acrylamide, le méthacrylamide, les alkyl- ou dialkyl(méth)acrylamides, les alkyl(méth)-acrylates, le vinylcaprolactone, le vinylpyrrolidone ; ou biens d'autres monomères tels que les esters vinyliques, l'alcool vinylique, l'anhydride maléique, le propylène-glycol, l'éthylène glycol. Les groupes alkyls ou dialkyls des fonctions amines ou ammoniums sont de préférence en C₁-C₉ et plus préférentiellement en C₁-C₃.

Les amines peuvent être primaires, secondaires ou tertiaires. Les amines secondaires et tertiaires sont préférentielles.

Les monomères vinyliques amino-substitués peuvent être polymérisés sous leur forme amine puis éventuellement quaternisés. Les amines peuvent être également quaternisées après la formation du polymère. Par exemple, les fonctions amines tertiaires peuvent être quatérnisées par réaction avec un sel de formule R'X où R' est un radical alkyle de chaîne courte (en C₁-C₇ de préférence et plus particulièrement en C₁-C₃) et X est un anion formant un sel hydrosoluble avec l'ammonium quaternaire.

Parmi les monomères vinyliques à fonction amines ou ammoniums quaternaires, on peut citer par exemple des composés vinyliques substitués par un groupe du type dialkylaminoalkyl (méth)acrylate, monoalkyl-aminoalkyl (méth)acrylate ; des sels de trialkyl-méthacryloxyalkyl ammonium ; des sels diallyliques d'ammonium quaternaire ; des monomères vinyliques quaternaires ayant des cycles portant des atomes d'azote tels que pyridinium, imidazolium, pyrrolidone quaternisée comme alkylvinylimidazolium, alkyl-vinylpyridinium, les sels quaternaires d'alkylvinyl pyrrolidone. Les portions alkyls de ces monomères sont de préférence des alkyls en C₁-C₃ et plus préférentiellement des alkyls en C₁ ou C₂.

On peut également citer comme monomères vinyliques amino-substitués, les dialkylaminoalkyl (méth)acrylates, les dialkylaminoalkyl (méth)acrylamides. Les groupes alkyls ou dialkyls sont de préférence en C₁-C₉ et plus préférentiellement en C₁-C₃.

Les polymères cationiques de l'invention peuvent comprendre des mélanges de monomères vinyliques dérivés d'amines et/ou de monomères vinyliques dérivés d'ammoniums quaternaires et/ou d'autres monomères compatibles. On peut citer, à titre d'exemple :
- les copolymères de 1-vinylpyrrolidone et de sel de 1-vinyl-3-méthylimidazolium (chlorure par exemple) (appelé Polyquaternium-16 dans le CTFA) tels que ceux vendus sous le nom LUVIQUAT par la société BASF ;
- les copolymères de 1-vinyl-2-pyrrolidone et de diméthylaminoéthylméthacrylate (appelés Polyquaternium-11 selon le CTFA) tels que ceux vendus sous le nom GAFQUAT (par exemple le GAFQUAT 755N) par la société GAF CORPORATION ;
- les homopolymères de chlorure de diméthyl-diallylammonium (Polyquaternium 5 selon le CTFA) et les copolymères d'acrylamide et de chlorure de diméthyl-diallylammonium (Polyquaternium-7 selon le CTFA) tels que ceux vendus sous le nom MERQUAT 550 et MERQUAT S par la Société MERCK ;
- les sels d'acides minéraux d'aminoalkylesters d'homo et de copolymères d'acides carboxyliques insaturés ayant de 3 à 5 atomes de carbone tels que ceux décrits dans le brevet USP 4 009 256.

Parmi les polymères cationiques utilisables, on peut citer aussi les polysaccharides cationiques tels que les dérivés de cellulose cationiques et les dérivés de l'amidon cationiques.

Parmi les polysaccharides cationiques, on peut citer les polymères de formule : 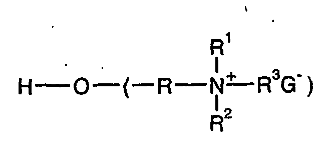 où :
H est un reste d'anhydroglucose tel que l'amidon ou un reste d'anhydroglucose cellulosique ;
R est un alkylène, un oxyalkylène, un polyoxyalkylène ou un hydroxyalkylène ou leurs mélanges ;
R¹, R² et R³, identiques ou différents, désignent un groupe alkyle, aryle, alkylaryle, arylalkyle, alkoxyalkyle ou alkoxyaryle ; chaque groupe contenant jusqu'à 18 atomes de carbone et le nombre total d'atomes de carbone par unité cationique est de préférence inférieure ou égale à 20.
G⁻est un anion résultant de la quaternisation de l'amine NR¹R²R³.

Parmi les polymères cellulosiques cationiques, on peut citer ceux vendus par la société AMERCHOL CORP. sous les noms JR et LR tels que les sels d'hydroxyéthylcellulose quaternaires obtenus par réaction avec un époxyde susbtitué par un triméthylammonium (polyquaternium-10 selon le CTFA). On peut citer également les sels d'hydroxyéthyl-cellulose quaternaires obtenus par réaction avec un époxyde substitué par le lauryl-diméthylamrnonium (polyquaternium-24 selon le CTFA) comme ceux vendus sous le nom POLYMER LM200 par AMERCHOL CORP.

On peut citer également, comme polymères cationiques utilisables selon l'invention, les dérivés de gomme de guar cationiques tels que le chlorure d'hydroxypropyltrimonium de guar vendu sous les noms JAGUAR par la société CELANESE CORP.

On peut citer également les éthers celluloses quaternaires telles que celles décrites dans le brevet USP. 3 962 418 et les copolymères de cellulose éthérifiés et d'amidon tels que ceux décrits dans le brevet US 3 958 581.

Les agents de conditionnement sont présents, dans les compositions selon l'invention, dans des proportions allant, de préférence, de 0,01 à 5 % en poids et de préférence de 0,1 à 3 % en poids par rapport au poids total de la composition.

### COMPOSITION

Le milieu cosmétiquement acceptable peut être constitué uniquement par de l'eau ou par un mélange d'eau et d'un ou plusieurs solvants cosmétiquement acceptables ou par un ou plusieurs solvants cosmétiquement acceptables, tel qu'un alcool inférieur en C₁-C₄, comme l'éthanol, l'isopropanol, le tertiobutanol, le n-butanol; les alkylèneglycols comme le propylèneglycol, les éthers de glycols.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir, de préférence, jusqu'à 5 % d'agents nacrants ou opacifiants bien connus dans l'état de la technique tels que par exemple les alcools gras supérieurs à C16, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir, en outre, au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les agents tensioactifs autres que ceux de l'invention, les polymères anioniques, non ioniques, cationiques ou amphotères, les protéines, les hydrolysats de protéines, les hydroxyacides, les vitamines, les provitamines tels que le panthénol, les silicones volatiles ou non, linéaires ou cycliques, réticulées ou non, organomodifiées ou non, non cationiques.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas ou substantiellement pas, altérées par la ou les adjonctions envisagées.

Ces additifs sont, éventuellement, présents dans la composition selon l'invention dans des proportions pouvant aller de 0,00001 à 20% en poids par rapport au poids total de la composition. La quantité précise de chaque additif est déterminée facilement par l'homme du métier selon sa nature et sa fonction.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des matières kératiniques, telles que notamment les cheveux, consistant à appliquer une composition telle que définie ci-dessus, puis à effectuer un rinçage à l'eau après un éventuel temps de pause.

Les compositions selon l'invention sont utilisées, de préférence, comme shampooings pour le lavage et le conditionnement des cheveux et ils sont appliqués dans ce cas-là sur les cheveux humides dans des quantités efficaces pour les laver, cette application étant suivie d'un rinçage à l'eau.

Les compositions conformes à l'invention sont également utilisables comme gel-douche pour le lavage et le conditionnement des cheveux et/ou de la peau, auquel cas ils sont appliqués sur la peau et/ou les cheveux humides et sont rincés après application.

Les compositions de l'invention peuvent également être utilisées en non rincé, et en particulier dans des lotions, dans des gels, dans des mousses, dans des aérosols.

Des exemples concrets, mais nullement limitatif, illustrant l'invention vont maintenant être donnés.

### EXEMPLES :

On réalise les deux compositions ci-après:

| Shampooing | |
|---|---|
| Lauryl sulfate de triéthanolamine à 40 % m.a (1) | 31,3 % |
| Copolymère (2) | 3,1 % |
| Carbonate de calcium en poudre (3) | 3 % |
| Qsp pH=7 | |
| Qsp eau = 100 | |

| Soin | |
|---|---|
| Carbonate de calcium en poudre (3) | 3 % |
| Ester quaternaire simple chaîne (4) | 4,5 % m.a. |
| Qsp pH=4 | |
| Qsp eau = 100 | |

| | |
|---|---|
| (1) en solution aqueuse (2) chlorure de diméthyl diallyl ammonium/acrylamide 50/50 en solution aqueuse à 8 % protégée (3) OMYAPUR 35 commercialisé par OMYA (4) Chlorure de behenyloxyhydroxypropyl triméthylammonium à 70 % dans l'eau / hexylèneglycol commercialisé par CHEMY sous l'appellation AKYPO 131 | |

La composition présente une texture agréable lors de l'application sur des cheveux humides. Sa rinçabilité est bonne. Les cheveux mouillés ne sont pas chargés et la mise en forme est aisée.

## Revendications

1. Composition cosmétique, notamment capillaire, comprenant, dans un milieu cosmétiquement acceptable :
- des particules solides contenant au moins 10 % en poids de carbonate de calcium ;
- au moins un agent de conditionnement choisi parmi :
(i)les tensioactifs cationiques choisis parmi les esters quaternaires, les diammonium quaternaires sauf ceux dérivés du diamino-propane, les imidazolinium et les amides,
(ii)les silicones cationiques, et
(iii) les polymères cationiques dont la densité de charge cationique est inférieure ou égale à 7 meq/g et, de préférence, supérieure ou égale à 0,05 meq/g.

2. Composition selon la revendication 1, **caractérisée par le fait que** les particules contenant au moins 10 % en poids de carbonate de calcium présentent une taille primaire moyenne en nombre comprise entre 2 nm et 2 µm, plus préférentiellement entre 5 et 500 nm, et plus préférentiellement encore entre 10 et 250 nm.

3. Composition selon la revendication selon l'une quelconque des revendications précédentes, **caractérisée par le fait que** les particules contiennent au moins 50 % en poids de carbonate de calcium, mieux encore au moins 70 % en poids, et encore mieux plus de 90 % en poids de carbonate de calcium.

4. Composition selon l'une quelconque des revendications précédentes, **caractérisée par le fait que** les particules sont des particules de carbonate de calcium substantiellement pur.

5. Composition selon l'une quelconque des revendications précédentes, **caractérisée par le fait que** les particules contenant du carbonate de calcium sont utilisées en une quantité comprise entre 0,01 % et 30 % en poids, et de préférence entre 0,05 % et 5 % en poids par rapport au poids total de la composition.

6. Composition selon l'une quelconque des revendications 1 à 5, **caractérisée par le fait que** le ou lesdits agents de conditionnement sont présents dans des proportions allant de 0,01 à 5 % en poids et, de préférence, de 0,1 à 3 % en poids par rapport au poids total de la composition.

7. Composition selon l'une quelconque des revendications précédentes, **caractérisée par le fait que** les sels de diammonium quaternaire présentent la formule (III) : 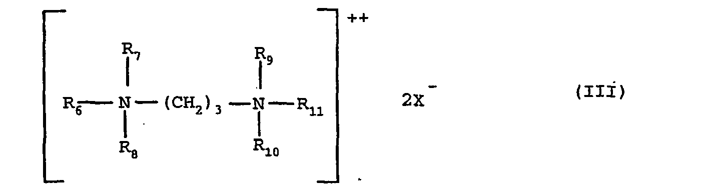 dans laquelle R₆ désigne un radical aliphatique comportant de 16 à 22 atomes de carbone, R₇, R₈, R₉, R₁₀, et R₁₁ sont choisis parmi l'hydrogène ou un radical alkyle comportant de 1 à 4 atomes de carbone, et X est un anion choisi dans le groupe des halogénures, acétates, phosphates, et sulfates.

8. Composition selon l'une quelconque des revendications 1 à 6, **caractérisée par le fait que** les sels d'ammonium quaternaire de l'imidazolinium présentent la formule (II) suivante : 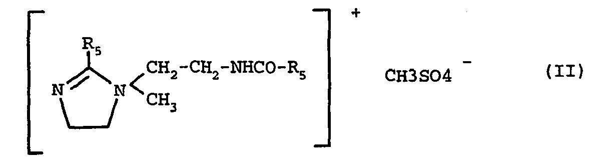 dans laquelle R₅ représente un mélange de radicaux alcényle et/ou alkyle comportant de 13 à 21 atomes de carbone et dérivés des acides gras du suif.

9. Composition selon l'une quelconque des revendications 1 à 6, **caractérisée par le fait que** les amides présentant la formule générale (I) suivante : 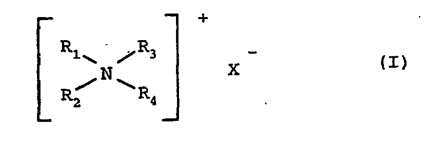 dans laquelle les radicaux R₁ à R₄, qui peuvent être identiques ou différents, représentent un radical alkylamide, comportant environ de 12 à environ 22 atomes de carbone; X est un anion choisi dans le groupe des halogénures, phosphates, acétates, lactates, alkylsulfates, alkyl-ou-alkylarylsulfonates.

10. Composition selon l'une quelconque des revendications 1 à 6, **caractérisée par le fait que** le polymère cationique est choisi parmi :
- les copolymères de monomères vinyliques ayant des fonctions amines ou ammoniums quaternaires avec des monomères à insaturation éthylénique hydrosolubles ;
- les copolymères de 1-vinylpyrrolidone et de sel de 1-vinyl-3-méthylimidazolium ;
- les copolymères de 1-vinyl-2-pyrrolidone et de diméthylaminoéthylméthacrylate;
- les homopolymères de chlorure de diméthyl-diallylammonium ;
- les copolymères d'acrylamide et de chlorure de diméthyl-diallylammonium ;
- les polysaccharides cationiques .

11. Composition selon l'une quelconque des revendications 1 à 6, **caractérisée par le fait que** la silicone cationique est une choisie parmi les «Amodiméthicone» et « Triméthylsilylamodiméthicone ».

12. Composition selon l'une quelconque des revendications précédentes, **caractérisée par le fait qu'**elle comprend, en outre, au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les agents tensioactifs autres que ceux de l'invention, les polymères anioniques, non ioniques, cationiques ou amphotères, les protéines, les hydrolysats de protéines, les hydroxyacides, les vitamines, les provitamines tels que le panthénol, les silicones volatiles ou non, linéaires ou cycliques, réticulées ou non, organomodifiées ou non, non cationiques.

13. Utilisation d'une composition telle définie dans l'une quelconque des revendications 1 à 12 comme shampooing.

14. Procédé de lavage et de conditionnement des cheveux consistant à appliquer une quantité efficace d'une composition telle que définie à l'une quelconque des revendications 1 à 12, puis à effectuer un rinçage à l'eau après un éventuel temps de pose.

## Claims

1. Cosmetic composition, especially a hair composition, comprising, in a cosmetically acceptable medium:
- solid particles containing at least 10% by weight of calcium carbonate;
- at least one conditioner chosen from:
(i) cationic surfactants chosen from quaternary esters, diquaternary ammoniums except for those derived from diaminopropane, imidazoliniums and amides,
(ii) cationic silicones, and
(iii) cationic polymers whose cationic charge density is less than or equal to 7 meq/g and preferably greater than or equal to 0.05 meq/g.

2. Composition according to Claim 1, **characterized in that** the particles containing at least 10% by weight of calcium carbonate have a number-average primary size of between 2 nm and 2 µm, more preferably between 5 and 500 nm and even more preferably between 10 and 250 nm.

3. Composition according to either of the preceding claims, **characterized in that** the particles contain at least 50% by weight of calcium carbonate, better still at least 70% by weight and even better still more than 90% by weight of calcium carbonate.

4. Composition according to any one of the preceding claims, **characterized in that** the particles are particles of substantially pure calcium carbonate.

5. Composition according to any one of the preceding claims, **characterized in that** the particles containing calcium carbonate are used in an amount of between 0.01% and 30% by weight and preferably between 0.05% and 5% by weight relative to the total weight of the composition.

6. Composition according to any one of Claims 1 to 5, **characterized in that** said conditioner(s) is (are) present in proportions ranging from 0.01% to 5% by weight and preferably from 0.1% to 3% by weight relative to the total weight of the composition.

7. Composition according to any one of the preceding claims, **characterized in that** the diquaternary ammonium salts have the formula (III): 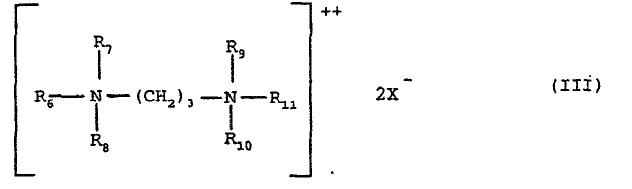 in which R₆ denotes an aliphatic radical containing from 16 to 22 carbon atoms, R₇, R₈, R₉, R₁₀ and R₁₁ are chosen from hydrogen or an alkyl radical containing from 1 to 4 carbon atoms, and X is an anion chosen from the group of halides, acetates, phosphates and sulfates.

8. Composition according to any one of Claims 1 to 6, **characterized in that** the quaternary ammonium salts of imidazolinium have the formula (II) below: 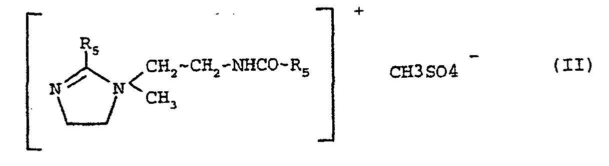 in which R₅ represents a mixture of alkenyl and/or alkyl radicals containing from 13 to 21 carbon atoms and tallow fatty acid derivatives.

9. Composition according to any one of Claims 1 to 6, **characterized in that** the amides have the general formula (I) below: 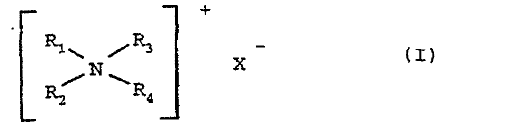 in which the radicals R₁ to R₄, which may be identical or different, represent an alkylamide radical containing from about 12 to about 22 carbon atoms; X is an anion chosen from the group of halides, phosphates, acetates, lactates, alkyl sulfates, alkyl sulfonates and alkylaryl sulfonates.

10. Composition according to any one of Claims 1 to 6, **characterized in that** the cationic polymer is chosen from:
- copolymers of vinyl monomers containing amine or quaternary ammonium functions with water-soluble ethylenically unsaturated monomers;
- copolymers of 1-vinylpyrrolidone and of a 1-vinyl-3-methylimidazolium salt;
- copolymers of 1-vinyl-2-pyrrolidone and of dimethylaminoethyl methacrylate;
- dimethyldiallylammonium chloride homopolymers;
- copolymers of acrylamide and of dimethyldiallylammonium chloride;
- cationic polysaccharides.

11. Composition according to any one of Claims 1 to 6, **characterized in that** the cationic silicone is chosen from "Amodimethicone" and "Trimethylsilylamodimethicone".

12. Composition according to any one of the preceding claims, **characterized in that** it also comprises at least one additive chosen from foam synergists such as C₁₀-C₁₈ 1,2-alkanediols or fatty alkanolamides derived from monoethanolamine or from diethanolamine, silicone or nonsilicone sunscreens, surfactants other than those of the invention, anionic, nonionic, cationic or amphoteric polymers, proteins, protein hydrolysates, hydroxy acids, vitamins, provitamins such as panthenol, and volatile or nonvolatile, linear or cyclic, crosslinked or noncrosslinked, organomodified or non-organomodified, non-cationic silicones.

13. Use of a composition as defined in any one of Claims 1 to 12, as a shampoo.

14. Process for washing and conditioning the hair, which consists in applying an effective amount of a composition as defined in any one of Claims 1 to 12, and then in rinsing with water after an optional leave-in time.

## Patentansprüche

1. Kosmetische Zusammensetzung und insbesondere haarkosmetische Zusammensetzung, die in einem kosmetisch akzeptablen Medium enthält:
- feste Partikel, die mindestens 10 Gew.-% Calciumcarbonat enthalten;
- mindestens ein Konditioniermittel, das ausgewählt ist unter:
(i) kationischen grenzflächenaktiven Stoffen, die unter den quartären Estern, quartären Diammoniumverbindungen, außer solchen, die von Diaminopropan abgeleitet sind, I-midazoliniumverbindungen und Amiden ausgewählt sind;
(ii) kationischen Siliconen, und
(iii) kationischen Polymeren, deren kationische Ladungsdichte 7 meq/ g beträgt oder darunter liegt und vorzugsweise 0,05 meq/g beträgt oder darüber liegt.

2. Zusammensetzung nach Anspruch 1, **dadurch gekennzeichnet, dass** die Partikel, die mindestens 10 Gew.-% Calciumcarbonat enthalten, eine zahlenmittlere Primärgröße von 2 nm bis 2 µm, besonders bevorzugt 5 bis 500 nm und ganz besonders bevorzugt 10 bis 250 nm aufweisen.

3. Zusammensetzung nach einem der vorhergehenden Ansprüche, **dadurch gekennzeichnet, dass** die Partikel mindestens 50 Gew.-% Calciumcarbonat, besser mindestens 70 Gew.-% und noch besser mindestens 90 Gew.-% Calciumcarbonat enthalten.

4. Zusammensetzung nach einem der vorhergehenden Ansprüche, **dadurch gekennzeichnet, dass** die Partikel im Wesentlichen reine Calciumcarbonatpartikel sind.

5. Zusammensetzung nach einem der vorhergehenden Ansprüche, **dadurch gekennzeichnet, dass** die Partikel, die Calciumcarbonat enthalten, in einer Menge von 0,01 bis 30 Gew.-% und vorzugsweise 0,05 bis 5 Gew.-%, bezogen auf das Gesamtgewicht der Zusammensetzung, verwendet werden.

6. Zusammensetzung nach einem der Ansprüche 1 bis 5, **dadurch gekennzeichnet, dass** das oder die Konditioniermittel in Mengenanteilen von 0,01 bis 5 Gew.-% und vorzugsweise 0,1 bis 3 Gew.-%, bezogen auf das Gesamtgewicht der Zusammensetzung, enthalten sind.

7. Zusammensetzung nach einem der vorhergehenden Ansprüche, **dadurch gekennzeichnet, dass** die quartären Diammoniumsalze die Formel (III) aufweisen: 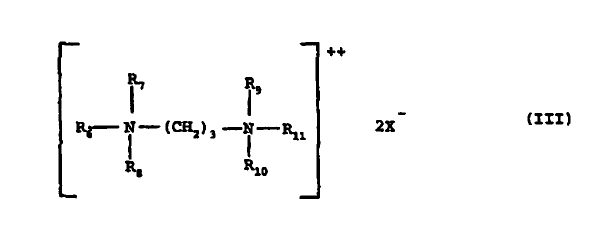 worin R₆ eine aliphatische Gruppe mit 16 bis 22 Kohlenstoffatomen ist, R₇, R₈, R₉, R₁₀ und R₁₁ unter Wasserstoff oder Alkylgruppen mit 1 bis 4 Kohlenstoffatomen ausgewählt sind und X ein Anion ist, das unter den Halogeniden, Acetaten, Phosphaten und Sulfaten ausgewählt ist.

8. Zusammensetzung nach einem der Ansprüche 1 bis 6, **dadurch gekennzeichnet, dass** die quartären Ammoniumsalze von Imidazolinium die folgende Formel (II) aufweisen: 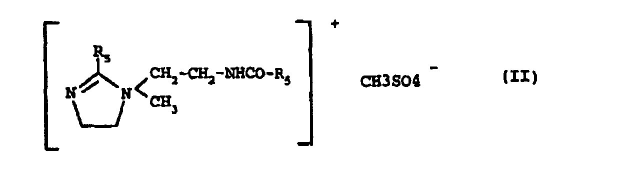 worin R₅ ein Gemisch von Alkenylgruppen und/oder Alkylgruppen mit 13 bis 21 Kohlenstoffatomen bedeutet, die von Talgfettsäuren abgeleitet sind.

9. Zusammensetzung nach einem der Ansprüche 1 bis 6, **dadurch gekennzeichnet, dass** die Amide die folgende allgemeine Formel (I) aufweisen: 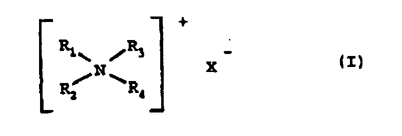 worin die Gruppen R₁ bis R₄, die gleich oder verschieden sein können, eine Alkylamidgruppe mit etwa 12 bis etwa 22 Kohlenstoffatomen bedeuten; und X ein Anion ist, das unter den Halogeniden, Phosphaten, Acetaten, Lactaten, Alkylsulfaten, Alkyl-oder Alkylarylsulfonaten ausgewählt ist.

10. Zusammensetzung nach einem der Ansprüche 1 bis 6, **dadurch gekennzeichnet, dass** das kationische Polymer ausgewählt ist unter:
- Copolymeren von Vinylmonomeren, die Aminofunktionen oder quartäre Ammoniumfunktionen aufweisen, und wasserlöslichen Monomeren mit ethylenisch ungesättigter Bindung;
- Copolymeren von 1-Vinylpyrrolidon und einem 1-Vinyl-3-methylimidazoliumsalz;
- Copolymeren von 1-Vinyl-2-pyrrolidon und Dimethylaminoethylmethacrylat;
- Homopolymeren von Dimethyldiallylammoniumchlorid;
- Copolymeren von Acrylamid und Dimethyldiallylammoniumchlorid;
- kationischen Polysacchariden.

11. Zusammensetzung nach einem der Ansprüche 1 bis 6, **dadurch gekennzeichnet, dass** das kationische Silicon unter den «Amodimethiconen» und «Trimethylsilylamodimethiconen» ausgewählt ist.

12. Zusammensetzung nach einem der vorhergehenden Ansprüche, **dadurch gekennzeichnet, dass** sie ferner mindestens einen Zusatzstoff enthält, der unter den Schaumsynergisten wie 1,2-Alcandiolen mit 10 bis 18 Kohlenstoffatomen oder von Mono- oder Diethanolamin abgeleiteten Alkanolfettamiden, siliconierten oder nicht siliconierten Sonnenschutzfiltern, grenzflächenaktiven Stoffen, die von den erfindungsgemäßen verschieden sind, anionischen, nichtionischen, kationischen oder amphoteren Polymeren, Proteinen, Proteinhydrolysaten, Hydroxysäuren, Vitaminen, Provitaminen, wie Panthenol, flüchtigen oder nicht flüchtigen , geradkettigen oder cyclischen, vernetzten oder nicht vernetzten, organomodifizierten oder nicht organomodifizierten, nicht kationischen Siliconen ausgewählt ist.

13. Verwendung einer Zusammensetzung nach einem der Ansprüche 1 bis 12 als Haarwaschmittel.

14. Verfahren zur Reinigung und zum Konditionieren von Haaren, das darin besteht, eine wirksame Menge einer Zusammensetzung nach einem der Ansprüche 1 bis 12 aufzutragen und nach einer gegebenenfalls abgewarteten Einwirkzeit mit Wasser zu spülen.
